# FASCICULE DE BREVET EUROPEEN

(11) **EP 4 536 328 B1**
(45) Date de publication et mention de la délivrance du brevet: **25.03.2026**
(21) Numéro de dépôt: 23731678.1
(22) Date de dépôt: 09.06.2023
(51) Int. Cl.: A61M 15/08, B05B 11/10

(54) **ENSEMBLE DE CONTRÔLE DE LA DISTRIBUTION DE PRODUIT PAR VOIE NASALE, SYSTÈME DE DISTRIBUTION ET PROCÉDÉ DE CONTRÔLE ASSOCIÉS**
ANORDNUNG ZUR STEUERUNG DER NASALEN ABGABE EINES PRODUKTS UND ZUGEHÖRIGES ABGABESYSTEM UND STEUERUNGSVERFAHREN
ASSEMBLY FOR CONTROLLING THE NASAL DELIVERY OF A PRODUCT, AND ASSOCIATED DELIVERY SYSTEM AND CONTROL METHOD

(30) Priorité: 10.06.2022 FR 2205636
(43) Date de publication de la demande: 16.04.2025
(73) Titulaire: NEMERA LA VERPILLIERE, 38290 La Verpillière (FR)
(72) Inventeur: REGARD, Alain, 01700 BEYNOST (FR); JOURDAN, Xavier, 38490 LE PASSAGE (FR); GUIMARD, Lenaic, 38850 CHARAVINES (FR); DECOCK, Thierry, 69002 LYON (FR)
(74) Mandataire: LLR
(86) Numéro de dépôt international: PCT/EP2023/065442
(87) Numéro de publication internationale: WO 2023/237715

(56) Documents cités:
- EP-A1- 3 749 400
- WO-A1-2018/109409
- WO-A1-2021/014076
- DE-U1- 202016 003 139

## Description

L'invention concerne le domaine technique de distribution de produit par voie nasale, plus précisément dans la cavité nasale, tout particulièrement à proximité de la zone olfactive disposée sur la face supérieure de la cavité nasale, au voisinage du cerveau.

On connaît déjà dans l'état la technique, notamment d'après le document WO 2021/014076 A1, un ensemble de contrôle de la distribution de produit comprenant un appareil mobile distant fixé sur l'oreille de l'utilisateur et un capteur d'orientation associé à un dispositif de distribution par voie nasale et dont un des objectifs est de déterminer la position du dispositif de distribution par voie nasale par rapport à l'appareil mobile distant. Selon ce document, l'appareil mobile comprend également un capteur d'orientation pour déterminer sa position dans l'espace. Toutefois, l'appareil mobile que propose ce document se fixe sur l'oreille, dont le pavillon peut présenter des dimensions différentes d'un utilisateur à un autre. Ainsi, la fixation de l'appareil mobile sur l'oreille de l'utilisateur peut poser des difficultés de stabilité et de répétabilité. En effet, la position de cet appareil mobile peut varier d'une utilisation à une autre, donc la position de cet appareil mobile par rapport au nez de l'utilisateur peut varier aussi, de sorte qu'il existe un risque non négligeable que le dispositif de distribution par voie nasale ne soit pas positionné par rapport au nez de l'utilisateur selon les angles optimaux qui permettraient une meilleure distribution de produit dans une zone cible, par exemple éloignée et difficile d'accès telle que la zone olfactive de la cavité nasale.

L'invention a notamment pour but d'optimiser, lors de son utilisation, la détermination de l'orientation du dispositif de distribution de produit par voie nasale par rapport au nez de l'utilisateur en vue notamment d'optimiser le positionnement du dispositif de distribution afin d'assurer une distribution de produit efficace.

Ce bon positionnement est particulièrement important dans le cas de distribution de médicaments visant à soigner notamment des maladies de type Alzheimer, Parkinson, ou encore des troubles de l'autisme, de l'humeur ou encore de la satiété.

A cet effet l'invention a pour objet un ensemble de contrôle de la distribution de produit dans une cavité nasale d'un utilisateur comprenant :
- un premier support configuré pour être fixé à un dispositif de distribution de produit par voie nasale et comprenant un premier capteur d'orientation configuré pour mesurer une donnée relative à l'orientation du premier support dans l'espace,
- un deuxième support configuré pour être fixé sur la tête de l'utilisateur et comprenant un deuxième capteur d'orientation configuré pour mesurer une donnée relative à l'orientation du deuxième support dans l'espace suivant trois axes de référence, et
- un système de traitement d'information configuré pour recevoir les données mesurées par le premier capteur d'orientation et le deuxième capteur d'orientation et pour déterminer une information sur l'orientation du premier support par rapport au deuxième support en fonction desdites données mesurées,
le deuxième support étant configuré pour être fixé sur le front ou le nez de l'utilisateur, de façon qu'un premier plan formé par deux des axes de référence du deuxième capteur d'orientation se trouve, lorsque le deuxième support est fixé sur la tête de l'utilisateur, dans une orientation prédéterminée par rapport à un plan sagittal de l'utilisateur, préférentiellement par rapport au plan sagittal de la tête de l'utilisateur, ou par rapport à un plan frontal de l'utilisateur, préférentiellement par rapport au plan frontal de la tête de l'utilisateur.

Le deuxième support est placé sur le front ou le nez de l'utilisateur, de sorte que le positionnement du deuxième capteur d'orientation soit fiable, stable et répétable lors des différentes utilisations. En effet, un tel agencement minimise la dépendance du positionnement ou de l'orientation du deuxième capteur à l'anatomie de l'utilisateur ou à la façon de le placer dans/sur une oreille de l'utilisateur.

La caractéristique « le deuxième support étant configuré pour être fixé sur le front ou le nez de l'utilisateur » inclut également le cas dans lequel le deuxième support est fixé à la fois sur le front et le nez de l'utilisateur.

On entend par orientation prédéterminée une orientation connue et sensiblement la même à chaque fois que le deuxième support est fixé sur le nez ou le front de l'utilisateur. C'est donc la structure du deuxième support et la façon dont il est fixé sur le front ou le nez de l'utilisateur, en suivant un guide d'utilisation par exemple, qui permet d'obtenir l'orientation prédéterminée. Il est ainsi possible de déduire de l'orientation du premier support par rapport au deuxième support, l'orientation du premier support par rapport au plan frontal ou sagittal de l'utilisateur puisque l'orientation du deuxième support par rapport au plan frontal ou sagittal est connue. En outre, la position du nez par rapport aux plans sagittal et frontal de l'utilisateur étant connue, il est ainsi possible de déterminer l'orientation du premier support et donc du dispositif de distribution dans la narine de l'utilisateur. La position du nez est fixe par rapport au plan frontal et sagittal de la tête de l'utilisateur.

On entend par « un premier support configuré pour être fixé à un dispositif de distribution de produit » un premier support adapté à être fixé solidaire en mouvement du dispositif de distribution produit. Un mouvement dans l'espace du dispositif de distribution de produit se traduit par un mouvement similaire du premier support et donc du premier capteur. Ainsi, l'orientation mesurée par le premier capteur fixer au premier support est représentative de l'orientation du dispositif de distribution. Toutefois, dans certains modes de réalisation, le premier support est fixé de manière amovible au dispositif de distribution de produit.

On entend par « un deuxième support configuré pour être fixé sur la tête de l'utilisateur » un deuxième support adapté à être fixé solidaire en mouvement de la tête de l'utilisateur. Un mouvement dans l'espace de la tête de l'utilisateur se traduit par un mouvement similaire du deuxième support et donc du deuxième capteur.

Le deuxième support permet d'indiquer l'orientation de la tête, donc du nez de l'utilisateur. On dispose ainsi d'un repère fiable et répétable pour détecter l'orientation du premier support par rapport au deuxième support, donc par rapport aux plans sagittal et/ou frontal de l'utilisateur, et préférentiellement aux plan sagittal et/ou frontal de la tête de l'utilisateur, puisque le deuxième support est monté selon une orientation précise sur la tête de l'utilisateur. Comme le premier support est configuré pour être fixé au dispositif de distribution, l'orientation du premier support représente celle du dispositif de distribution. Ainsi l'orientation du dispositif de distribution dans la narine de l'utilisateur peut être déterminée de manière plus fiable et plus précise.

Il est particulièrement avantageux que le deuxième support soit fixé sur le front ou le nez de l'utilisateur plutôt que dans ou sur une oreille. En effet, l'oreille est un organe souple et par conséquent la position et l'orientation d'un capteur dans/sur une oreille est moins fiable et répétable que sur le nez ou le front.

La détermination de l'information sur l'orientation du premier support par rapport au deuxième support et notamment la mesure des angles dits relatifs entre le premier capteur d'orientation et le deuxième capteur d'orientation est pertinente pour évaluer si le dispositif de distribution est correctement positionné dans une des narines avant l'activation de la distribution de produit. Plus particulièrement, le fait qu'un plan formé par deux axes de référence du deuxième capteur d'orientation soit, grâce à la structure du deuxième support et à son positionnement sur le front ou le nez de l'utilisateur par l'utilisateur lui-même ou par un praticien, agencé de manière prédéterminée, de préférence de façon parallèle, par rapport au plan sagittal ou frontal de l'utilisateur, préférentiellement par rapport au plan sagittal ou frontal de la tête de l'utilisateur, permet une mesure de l'orientation du dispositif de distribution plus facile, plus précise et avantageusement une orientation du dispositif de distribution lors de son utilisation particulièrement stable et répétable et, en particulier très peu dépendante de l'anatomie de l'utilisateur.

La détermination précise du positionnement du dispositif de distribution par rapport au nez présente plusieurs avantages. Cette détermination précise aide par exemple à identifier, lors des « études utilisateur » pendant la phase de développement d'un dispositif de distribution, le dispositif de distribution qui permet d'obtenir le positionnement requis pour une distribution de produit plus efficace dans la zone cible lors de l'activation du dispositif. Dans d'autres cadres d'utilisation, comme par exemple une étude clinique ou une utilisation à domicile, cela permet également de guider l'utilisateur afin qu'il positionne le dispositif de distribution selon les angles requis pour une distribution de produit plus efficace dans la zone cible. Ce bon positionnement est particulièrement important dans le cas de distribution de médicaments visant à soigner des maladies de type Alzheimer, Parkinson, ou encore des troubles de l'autisme, de l'humeur ou encore de la satiété.

Le plan sagittal d'un utilisateur correspond au plan qui divise le corps en une partie droite et une partie gauche. Le plan sagittal de la tête de l'utilisateur correspond au plan qui divise la tête en une partie droite et une partie gauche. Le plan frontal de l'utilisateur, également parfois appelé plan coronal, correspond à un plan perpendiculaire au plan sagittal de l'utilisateur et sépare le corps en une partie antérieure ou ventrale et une partie postérieure ou dorsale. Le plan frontal de la tête de l'utilisateur correspond à un plan perpendiculaire au plan sagittal de la tête de l'utilisateur et sépare la tête en une partie antérieure et une partie postérieure. La cloison nasale, séparant les deux narines d'un utilisateur se trouve sensiblement dans le plan sagittal.Le premier support comporte des moyens de fixation au dispositif de distribution qui peuvent être des moyens de fixation permanents ou amovibles. L'avantage de moyens de fixation amovibles réside dans le fait que l'ensemble de contrôle de la distribution de produit peut être utilisé pour contrôler l'orientation de différentes sortes de dispositifs de distribution qui comprennent, par exemple, des têtes de distribution différentes. L'ensemble de contrôle de la distribution de produit est donc de préférence réutilisable alors que le dispositif de distribution et notamment sa tête de distribution peuvent être à usage unique.

Le système de traitement d'informations peut communiquer avec le premier capteur et le deuxième capteur de façon filaire. Le premier capteur et le deuxième capteur peuvent également coopérer chacun avec le système de traitement via un module de communication sans fil. Le système de traitement peut être par exemple un smartphone ou un microcontrôleur, par exemple embarqué sur le deuxième support ou sur le premier support.

Le premier capteur d'orientation et le deuxième capteur d'orientation sont par exemple chacun une centrale inertielle comprenant au moins un accéléromètre et un gyroscope. Les trois axes de l'accéléromètre, également appelés axes de référence du capteur correspondant, forment avantageusement un repère orthonormé. Le gyroscope permet de mesurer la vitesse angulaire sur trois axes qui sont avantageusement les trois axes de l'accéléromètre de la même centrale inertielle. Éventuellement, la centrale inertielle comprend en outre un magnétomètre permettant d'améliorer la précision de mesure de la centrale inertielle.

Suivant d'autres caractéristiques optionnelles mais non obligatoires de l'invention, l'ensemble de contrôle de la distribution de produit comprend une ou plusieurs des caractéristiques suivantes, prises seules ou selon toute combinaison techniquement admissible :
- L'orientation prédéterminée du premier plan correspond à une orientation parallèle au plan sagittal de l'utilisateur ou au plan frontal de l'utilisateur.

Ainsi, grâce au deuxième support ainsi configuré, le deuxième capteur est monté fixe et de façon parallèle au plan sagittal ou au plan frontal sur la tête de l'utilisateur, de sorte que l'orientation du dispositif de distribution est connue en référence au plan sagittal ou frontal de l'utilisateur. On dispose ainsi d'un repère fiable et répétable pour détecter l'orientation du premier support par rapport au deuxième support et donc par rapport à la narine de l'utilisateur. En outre, la détermination de l'orientation du premier support par rapport au plan sagittal ou frontal de l'utilisateur et donc par rapport à la narine de l'utilisateur est simplifiée.
- L'orientation prédéterminée du premier plan correspond à une orientation parallèle au plan sagittal de la tête de l'utilisateur ou au plan frontal de la tête de l'utilisateur. On dispose ainsi d'un repère fixe par rapport à la narine de l'utilisateur quelle que soit l'orientation de la tête de l'utilisateur.
- Le deuxième support est configuré de sorte que, une fois fixé sur le front ou le nez de l'utilisateur, le deuxième capteur se trouve au-dessus du nez de l'utilisateur, entre les yeux ou au centre du front.
- Le deuxième support est configuré pour être fixé sur le front ou le nez de l'utilisateur, de façon que le premier plan se trouve dans une orientation prédéterminée par rapport au plan sagittal de l'utilisateur et qu'un deuxième plan, formé par deux des axes de référence du deuxième capteur d'orientation et différent du premier plan, se trouve dans une orientation prédéterminée par rapport au plan frontal de l'utilisateur.

Plus généralement l'utilisateur ou un praticien sont aptes à positionner le deuxième support sur le front ou le nez de l'utilisateur et la structure du deuxième support permet alors de positionner le deuxième support de sorte que le premier plan et le deuxième plan soient respectivement dans leur orientation prédéterminée par rapport aux plans sagittal et frontal de l'utilisateur, c'est à dire de préférence respectivement parallèle au plan sagittal et parallèle au plan frontal. Ceci permet d'obtenir un repère encore plus fiable et plus répétable pour détecter l'orientation du premier support par rapport au deuxième support, donc par rapport à la narine de l'utilisateur puisqu'on connait l'orientation du deuxième support à la fois par rapport au plan sagittal mais aussi par rapport au plan frontal.
- Le deuxième support est configuré pour être fixé sur le front ou le nez de l'utilisateur, de façon que le premier plan se trouve dans une orientation prédéterminée par rapport au plan sagittal de la tête de l'utilisateur et qu'un deuxième plan, formé par deux des axes de référence du deuxième capteur d'orientation et différent du premier plan, se trouve dans une orientation prédéterminée par rapport au plan frontal de la tête de l'utilisateur.

Ceci permet d'obtenir un repère indépendant de l'orientation de la tête de l'utilisateur.
- Le deuxième support est configuré pour être fixé à la fois sur le front et sur le nez de l'utilisateur. Cela augmente davantage encore l'immobilisation du deuxième support par rapport à la tête de l'utilisateur et donc la fiabilité et la répétabilité de la mesure de l'orientation du dispositif de distribution de produit.
- Le deuxième capteur d'orientation comprend un accéléromètre configuré pour mesurer l'accélération linéaire suivant trois axes de mesure, les axes de mesure correspondant aux axes de référence du deuxième capteur.

La détermination de l'orientation du premier capteur par rapport aux plans sagittal et/ou frontal de l'utilisateur, préférentiellement par rapport aux plans sagittal et/ou frontal de la tête de l'utilisateur, est ainsi simplifiée.
- Le deuxième support comprend un dispositif de fixation sur la tête de l'utilisateur, une embase de réception du deuxième capteur d'orientation, des éléments de réglage de la position de l'embase par rapport au dispositif de fixation et des éléments de verrouillage de la position de l'embase de sorte que la position du deuxième capteur d'orientation par rapport au plan sagittal de l'utilisateur, préférentiellement par rapport au plan sagittal de la tête de l'utilisateur, puisse être réglée et verrouillée une fois que le dispositif de fixation est fixé sur la tête d'un utilisateur, les éléments de réglage comprenant de préférence des éléments de pivotement de l'embase selon un axe de pivotement perpendiculaire au plan sagittal de l'utilisateur, préférentiellement au plan sagittal de la tête de l'utilisateur.

Le dispositif de fixation permet notamment de positionner le deuxième capteur de sorte que le premier plan soit dans l'orientation prédéterminée par rapport au plan sagittal de l'utilisateur, préférentiellement au plan sagittal de la tête de l'utilisateur, et de préférence parallèle au plan sagittal de l'utilisateur, préférentiellement au plan sagittal de la tête de l'utilisateur. Les éléments de réglage et de verrouillage permettent d'immobiliser le deuxième capteur par rapport au plan sagittal de l'utilisateur, préférentiellement au plan sagittal de la tête de l'utilisateur. Dans le cas où les éléments de réglage comprennent des éléments de pivotement de l'embase selon un axe de pivotement perpendiculaire au plan sagittal de l'utilisateur, préférentiellement au plan sagittal de la tête de l'utilisateur, les éléments de réglage permettent de positionner le deuxième capteur dans une position où un plan formé par deux axes de l'accéléromètre du deuxième capteur est parallèle au plan frontal de l'utilisateur, préférentiellement au plan frontal de la tête de l'utilisateur. Ceci permet d'avoir, en plus du plan sagittal de l'utilisateur, préférentiellement du plan sagittal de la tête de l'utilisateur, un deuxième plan de référence par rapport au nez de l'utilisateur.

En pratique et de façon avantageuse, lorsque le deuxième support est positionné sur le front ou le nez de l'utilisateur debout ou assis et qui regarde dans une direction horizontale, vers l'horizon, on peut faire pivoter l'embase du capteur de façon à ce que le deuxième support soit vertical par rapport au sol.

Ainsi, le deuxième capteur est lié en mouvement avec le nez de l'utilisateur. Ceci a pour avantage que la position du deuxième capteur représente en temps réel l'orientation du nez et notamment des plans sagittal et frontal de l'utilisateur, préférentiellement des plans sagittal et frontal de la tête de l'utilisateur, qui définissent une position de référence pour le premier capteur. Il est donc possible de calculer les angles relatifs entre le premier support et le nez, notamment entre le premier support et à la fois le plan frontal et le plan sagittal de l'utilisateur, préférentiellement entre le premier support et à la fois les plans frontal et sagittal de la tête de l'utilisateur, pour savoir de manière encore plus précise si le dispositif est correctement positionné.

On entend par « lié en mouvement » le fait qu'un mouvement dans l'espace du nez de l'utilisateur se traduit par un mouvement similaire du deuxième support et donc du deuxième capteur.
- Le deuxième support comprend un dispositif de fixation sur la tête de l'utilisateur et une embase de réception du deuxième capteur d'orientation, le dispositif de fixation comprenant au moins trois zones d'appui sur le visage de l'utilisateur.

Par exemple, le deuxième support est configuré pour être en contact avec le visage de l'utilisateur au moyen, par exemple, de deux appuis sur le nez et un appui sur le front, ou encore de deux appuis sur les oreilles et deux appuis sur le nez. De cette manière, la fixation du deuxième support sur le visage de l'utilisateur est plus stable et la surveillance du positionnement du dispositif de distribution par rapport au nez est plus précise.
- Le dispositif de fixation du deuxième support comprend des moyens de centrage sur le nez de l'utilisateur, par exemple des patins prenant appui de part et d'autre du nez ; et des branches configurées pour prendre appui sur les oreilles de l'utilisateur ou un élément de plaquage contre le front, par exemple un bandeau élastique configuré pour être plaqué autour de la tête de l'utilisateur. Le positionnement du deuxième support est ainsi particulièrement intuitif et stable.

Lorsque le deuxième support comprend un bandeau élastique configuré pour être plaqué autour de la tête de l'utilisateur, le deuxième support comprend de préférence une plaque plaquée contre le front et portant le deuxième capteur. Il s'agit d'un mode de fixation sur le front particulièrement efficace.
- Le premier capteur d'orientation est configuré pour mesurer une donnée relative à l'orientation du premier support dans l'espace suivant trois axes de référence, le premier support étant configuré pour que, lorsqu'il est fixé au dispositif de distribution, un des axes de référence du premier capteur d'orientation soit dans une orientation prédéterminée par rapport à un axe de distribution du dispositif de distribution, de préférence pour qu'un des axes de référence du premier capteur d'orientation soit parallèle, de préférence encore, confondu avec l'axe de distribution.

Ainsi, l'orientation donnée par le premier capteur d'orientation correspond à l'orientation exacte de l'axe de distribution, ce qui fournit une information précise sur l'orientation d'une tête de distribution du dispositif de distribution et simplifie le traitement de l'information.

La tête de distribution du dispositif de distribution définit un axe de distribution correspondant à l'axe dans lequel le produit est distribué. En général, mais pas exclusivement, l'axe de distribution est confondu avec un axe du réservoir de produit, notamment un axe de symétrie de ce réservoir.

Selon un exemple non limitatif, le premier support présente un axe de symétrie confondu avec l'axe de symétrie du dispositif de distribution lorsque le premier support est fixé au le dispositif de distribution, de telle sorte que l'un des axes de référence du premier capteur est dans une position prédéterminée par rapport à l'axe de distribution du dispositif, de préférence un des axes de référence du premier capteur est parallèle de préférence encore confondu à l'axe de distribution.
- Le premier support comprend des moyens d'activation configurés pour être actionnés par les doigts d'une personne et pour coopérer avec le dispositif de distribution afin de distribuer une dose de produit, le premier capteur d'orientation étant configuré pour détecter l'actionnement des moyens d'activation en plus de l'orientation du premier support.

L'actionnement des moyens d'activation entraîne un mouvement d'au moins une partie du dispositif de distribution, par exemple d'une première partie de pompe de distribution mobile par rapport à une deuxième partie de la pompe de distribution. La détection de l'actionnement des moyens d'activation permet de détecter le moment où a été effectuée l'administration, donc l'orientation du dispositif au moment de l'administration. Ceci permet de mesurer l'orientation du dispositif de distribution au moment de l'actionnement de la distribution de produit. Par exemple, le premier capteur d'orientation est agencé sur les moyens d'activation.

En variante ou en combinaison, le premier capteur d'orientation comprend un capteur de force apte à détecter l'actionnement des moyens d'activation du dispositif de distribution ou la prise en main du dispositif de distribution. Avantageusement le premier support comprend également un organe de contrôle, apte à déclencher l'activation de l'ensemble de contrôle après avoir détecté, au moyen du premier capteur, la prise en main du dispositif de distribution par un utilisateur.

Selon un exemple de réalisation de l'invention, les moyens d'activation du premier support sont configurés pour être déplacés dans une direction confondue avec la direction d'un axe du réservoir. Par exemple, l'axe du réservoir est un axe de symétrie du réservoir, notamment encore un axe du réservoir de produit passant par l'orifice de distribution.

Alternativement, les moyens d'activation du premier support sont configurés pour être déplacés selon une direction orthogonale par rapport à la direction d'un axe du réservoir. Par exemple, l'axe du réservoir est un axe de symétrie du réservoir, notamment encore un axe du réservoir de produit passant par l'orifice de distribution. Ainsi, on peut utiliser un dispositif à activation latérale.
- L'ensemble de contrôle de la distribution de produit comprend un moyen de signalisation, dit premier moyen de signalisation, configuré pour émettre un premier signal lorsque le système de traitement détermine que le premier support est positionné selon une orientation prédéterminée par rapport au deuxième support.

Ceci permet de fournir une information à l'utilisateur en temps réel sur la bonne orientation du dispositif de distribution, pour qu'il puisse actionner des moyens d'activation du dispositif de distribution bien orienté et qu'une quantité satisfaisante de produit soit distribuée dans la zone cible. Le signal peut être un signal visuel, sonore ou tactile ou encore l'une quelconque de leurs combinaisons. Par exemple, la signalisation peut être émise de façon visuelle sur un écran déporté.
- L'ensemble de contrôle de la distribution de produit comprend un moyen de signalisation, dit second moyen de signalisation, configuré pour émettre un second signal continu ou intermittent, aussi longtemps que le système de traitement détermine que le premier support n'est pas positionné selon une orientation prédéterminée par rapport au deuxième support.

Le second moyen de signalisation joue alors un rôle de guidage dans le positionnement du premier support afin que la distribution de produit ne soit déclenchée que lorsque le dispositif est correctement positionné. Le second moyen de signalisation peut être le même que le premier moyen de signalisation. Par exemple, il est possible d'utiliser un voyant lumineux capable d'émettre deux couleurs différentes selon les circonstances.
- Le système de traitement est configuré pour déterminer, lorsque le deuxième support est fixé sur la tête de l'utilisateur, en fonction des données mesurées par les premier et deuxième capteurs d'orientation : un premier angle (a) formé entre un axe de distribution du dispositif de distribution et le plan frontal de l'utilisateur, et/ou un deuxième angle (b) formé entre un axe de distribution du dispositif de distribution et le plan sagittal de l'utilisateur. L'orientation prédéterminée correspond à une position du premier support permettant que l'axe du dispositif de distribution, dit axe de distribution, forme avec le plan frontal de l'utilisateur un premier angle (a) compris entre 25° et 35°, de préférence égal à 30° ; et avec le plan sagittal de l'utilisateur un deuxième angle (b) compris entre 5° et 15°, de préférence égal à 10°.
- Le système de traitement est configuré pour déterminer, lorsque le deuxième support est fixé sur la tête de l'utilisateur, en fonction des données mesurées par les premier et deuxième capteurs d'orientation : un premier angle (α) formé entre un axe de distribution du dispositif de distribution et le plan frontal de la tête de l'utilisateur, et/ou un deuxième angle (β) formé entre un axe de distribution du dispositif de distribution et le plan sagittal de la tête de l'utilisateur.

De préférence, l'orientation prédéterminée correspond à une position du premier support permettant que l'axe du dispositif de distribution, dit axe de distribution, forme avec le plan frontal de la tête de l'utilisateur un premier angle (α) compris entre 25° et 35°, de préférence égal à 30° ; et avec le plan sagittal de la tête de l'utilisateur un deuxième angle (β) compris entre 5° et 15°, de préférence égal à 10°.

D'après les recherches et les essais décrits dans la littérature scientifique, une telle inclinaison dans le plan sagittal et le plan frontal de l'utilisateur, de préférence dans le plan sagittal et le plan frontal de la tête de l'utilisateur, par rapport au nez de l'utilisateur favorise la déposition optimale du produit dans certaines zones spécifiques, tout particulièrement la zone olfactive dans la cavité nasale supérieure.
- Avantageusement, les moyens d'activation sont configurés pour être verrouillés selon l'information fournie sur l'orientation du premier support par rapport au deuxième support.

Cette configuration permet de verrouiller les moyens d'activation lorsque le premier support n'est pas positionné selon une orientation prédéterminée. L'avantage de cette configuration est de favoriser une administration optimale de la dose de produit dans la zone cible. Cette configuration est encore plus intéressante lorsque le dispositif de distribution de produit contient une dose unique.
- Avantageusement, l'ensemble de contrôle de la distribution de produit comprend un capteur de contact apte à être agencé sur la tête de distribution et configuré pour détecter un contact avec une zone prédéterminée du visage de l'utilisateur

Ceci permet d'apporter une vérification supplémentaire du positionnement optimal du dispositif de distribution par rapport à une narine de l'utilisateur. En particulier, on peut prévoir de vérifier à la fois un contact avec une lèvre de l'utilisateur et le bas du nez de l'utilisateur, la lèvre pouvant être par exemple la lèvre supérieure ou la lèvre supérieure blanche, ce qui permet de favoriser un positionnement sagittal satisfaisant. Le capteur de contact peut être un capteur photosensible ou un capteur résistif ou un capteur capacitif ou un contacteur électrique.

L'invention a également pour objet un système de distribution de produit comprenant un dispositif de distribution, lequel comprend un réservoir de produit et une tête de distribution, et un ensemble de contrôle de la distribution de produit dans une cavité nasale d'un utilisateur tel que décrit ci-dessus.

Avantageusement, le dispositif de distribution est configuré pour être fixé au premier support via des moyens de fixation, tel qu'un système adhésif, un système de clips, un système d'encliquetage, une coopération de forme entre le premier support et le dispositif de distribution comme un emmanchement.

Avantageusement, le système de distribution de produit peut comprendre une partie jetable et une partie réutilisable. Dans ce cas, le premier support fait partie de la partie réutilisable et le réservoir de produit ou la tête de distribution peuvent faire partie de la partie jetable de manière à ce que différents types de produits ou de dispositifs de distribution puissent être utilisés avec le système.

L'invention a encore pour objet un procédé de contrôle de la distribution de produit dans une cavité nasale d'un utilisateur à l'aide d'un ensemble de contrôle de la distribution de produit tel que décrit précédemment, comprenant les étapes suivantes :
- étape 1: le premier support est fixé à un dispositif de distribution de produit par voie nasale,
- étape 2 : le deuxième support est fixé sur le front ou le nez d'un utilisateur de sorte qu'un premier plan formé par deux des axes de référence du deuxième capteur d'orientation se trouve dans une orientation prédéterminée par rapport à un plan sagittal de l'utilisateur, préférentiellement le plan sagittal de la tête de l'utilisateur, ou par rapport à un plan frontal de l'utilisateur, préférentiellement le plan frontal de la tête de l'utilisateur.

- étape 3 : le premier capteur d'orientation et le deuxième capteur d'orientation mesurent des données, et
- étape 4 : le système de traitement d'information détermine une information sur l'orientation du premier support par rapport au deuxième support en fonction des données mesurées par les premier et deuxième capteurs d'orientation.

Avantageusement, lors de l'étape 2 de fixation du deuxième support sur la tête de l'utilisateur, le premier plan est dans une orientation prédéterminée par rapport au plan sagittal de l'utilisateur, préférentiellement par rapport au plan sagittal de la tête de l'utilisateur, et un réglage du positionnement angulaire du deuxième capteur d'orientation est effectué de sorte qu'un deuxième plan, formé par deux des axes de référence du deuxième capteur d'orientation et différent du premier plan, se trouve dans une orientation prédéterminée par rapport à un plan frontal de l'utilisateur, préférentiellement par rapport à au plan frontal de la tête de l'utilisateur.

### Brève description des figures

L'invention sera mieux comprise à la lecture de la description suivante, donnée uniquement à titre d'exemple et faite en se référant aux dessins annexés dans lesquels :
[Fig. 1] la figure 1 est une vue de côté d'un dispositif de distribution de produit par voie nasale muni d'un premier support et d'un capteur faisant partie d'un ensemble de contrôle de la distribution de produit dans une cavité nasale d'un utilisateur selon l'invention :
[Fig. 2] la figure 2 est une vue en perspective d'un deuxième support selon un premier mode de réalisation, le deuxième support faisant partie d'un ensemble de contrôle de la distribution de produit dans une cavité nasale d'un utilisateur ;
[Fig. 3] la figure 3 est une vue de face du deuxième support selon un deuxième mode de réalisation ;
[Fig. 4] la figure 4 est une vue de côté du deuxième support de la figure 3, dans une orientation prédéterminée ;
[Fig. 5] la figure 5 est une vue de côté du deuxième support de la figure 3, dans une autre orientation prédéterminée ;
[Fig. 6] la figure 6 est une vue de côté d'un système de distribution de produit dans une cavité nasale d'un utilisateur comprenant le dispositif de distribution muni du premier support de la figure 1 et le deuxième support de la figure 2 ;
[Fig. 7] la figure 7 est une vue de face du système de distribution de produit de la figure 6 ;
[Fig. 8] la figure 8 est un ensemble de vues (figures 8a et 8b) du premier support de la figure 1 fixé au dispositif de distribution.

### Description détaillée

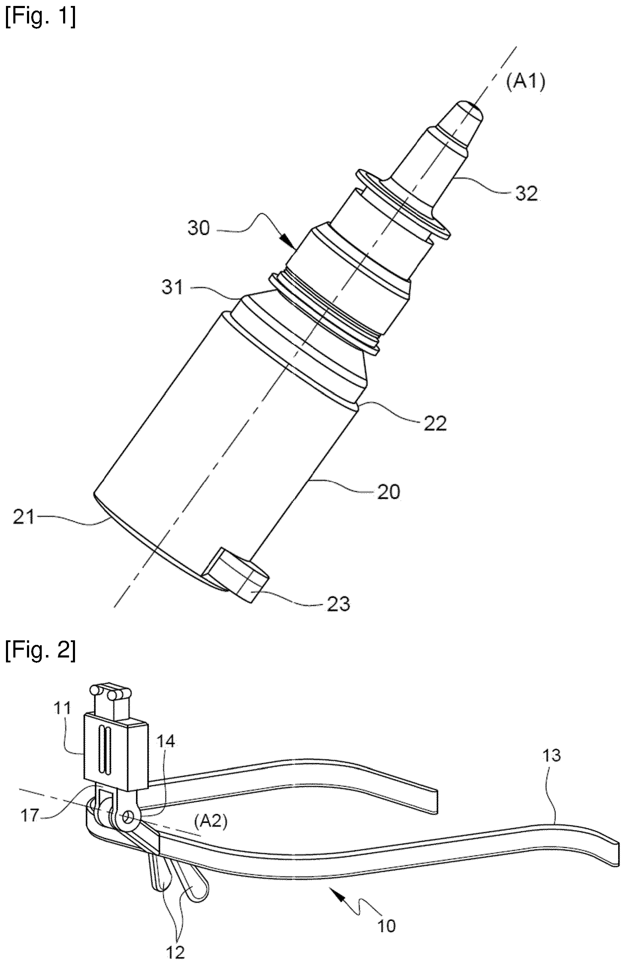

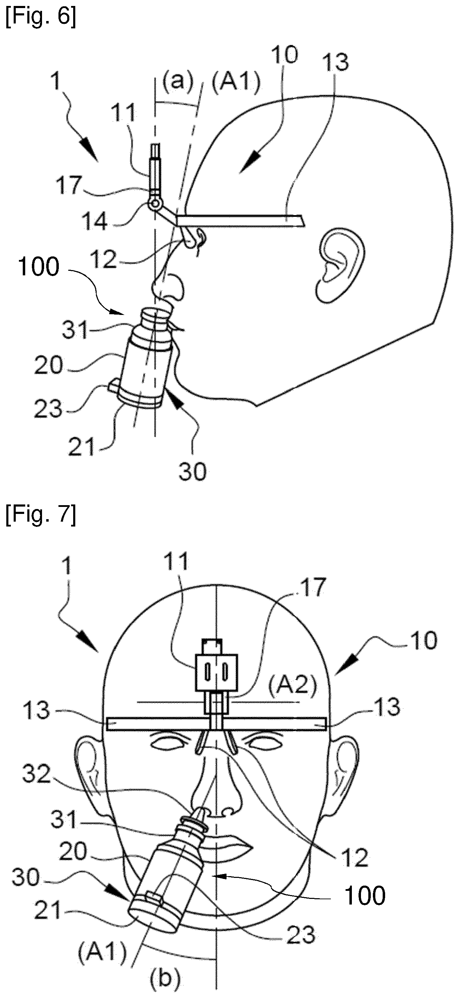

Les figures 1 à 2 représentent un ensemble de contrôle de la distribution de produit dans une cavité nasale d'un utilisateur selon un premier mode de réalisation. Cet ensemble, également illustré aux figures 6 et 7, est désigné par la référence générale 1 et est représenté associé au dispositif de distribution 30 de produit par voie nasale sur ces figures.

Plus généralement, on a représenté sur les figures 6 et 7, un système de distribution de produit 100 dans une cavité nasale d'un utilisateur comprenant le dispositif de distribution 30 et l'ensemble 1 de contrôle de la distribution de produit.

Tel qu'illustré par la figure 1, l'ensemble comprend un premier support 20, qui porte un premier capteur 21 d'orientation. Le premier support 20 est de préférence de forme générale cylindrique et creuse, délimitant un logement de réception pour le réservoir. Dans ce mode de réalisation, le fond du premier support porte le premier capteur 21 d'orientation, par exemple sur sa face extérieure. Le premier support 20 comprend des moyens de fixation 22 au dispositif de distribution 30 qui sont, par exemple, des moyens de fixation par encliquetage ou par coopération de forme.

Le premier capteur 21 d'orientation est configuré pour mesurer une donnée relative à l'orientation du premier support 20 dans l'espace suivant trois axes de référence.

Le premier capteur 21 d'orientation est configuré pour communiquer avec un système de traitement d'information afin de fournir, de préférence en temps réel, les données relatives à l'orientation du premier support 20 dans l'espace.

Le système de traitement appartient également à l'ensemble 1.

Tel que visible à la figure 1, le dispositif de distribution 30 peut comprendre un réservoir de produit 31 de forme cylindrique avec un axe de symétrie (A1). Un organe de distribution, tel qu'une pompe ou une valve, est assemblé sur le réservoir de produit 31. Le dispositif de distribution 30 comprend encore une tête de distribution 32 connectée à l'organe de distribution. La tête de distribution 32 présente un orifice de distribution qui est relié au réservoir de produit 31 par un conduit de passage du produit à distribuer. Dans l'exemple illustré, la tête de distribution 32 comprend un manchon creux formant un embout nasal et présentant un axe de symétrie confondu avec l'axe de symétrie (A1) du réservoir de produit 31 de sorte que l'axe de symétrie (A1) du réservoir de produit 31 est également l'axe du dispositif de distribution (A1). Par ailleurs, il est avantageux d'agencer l'orifice de distribution dans l'axe du dispositif de distribution (A1).

Le premier support 20 est configuré pour que, lorsqu'il est fixé au dispositif de distribution 30, un des axes de référence du premier capteur 21 d'orientation soit dans une orientation prédéterminée par rapport à l'axe de distribution (A1) du dispositif de distribution 30, de préférence un des axes de références du premier capteur 21 soit parallèle, de préférence encore confondu avec l'axe de distribution (A1).

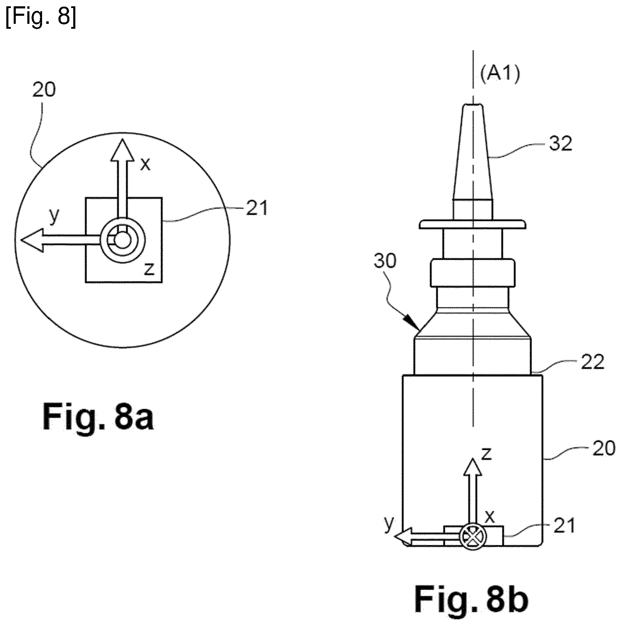

Selon un mode de réalisation du premier support 20 tel que visible aux figures 8a et 8b, le premier capteur 21 d'orientation est agencé à l'une des extrémités du premier support 20, par exemple à l'extrémité située à l'opposé de la tête de distribution 32. L'axe z du premier capteur 21 d'orientation coïncide avec l'axe de distribution du dispositif de distribution (A1) lorsque le premier support 20 est fixé au dispositif de distribution 30.

Le premier support 20 comprend en outre avantageusement des moyens d'activation 23 configurés pour être actionnés par les doigts d'une personne et pour coopérer avec le dispositif de distribution 30 afin de distribuer une dose prédéterminée de produit. Par exemple, les moyens d'activation 23 sont adaptés à déplacer le réservoir de produit 31 par rapport à la tête de distribution 32 selon l'axe de distribution (A1) pour actionner l'organe de distribution. De manière alternative, les moyens d'activation 23 pourraient être composés par le fond du premier support 20, la surface extérieure dudit fond formant une surface d'appui pour déplacer le réservoir de produit 31 par rapport à la tête de distribution 32 selon l'axe de distribution (A1).

Avantageusement, le premier capteur 21 d'orientation est apte à détecter l'actionnement des moyens d'activation 23. Ainsi, le premier capteur 21 d'orientation permet de détecter le moment où a été effectuée l'administration et l'orientation du dispositif au moment de l'administration. Le premier capteur 21 d'orientation est par exemple apte à être positionné sur les moyens d'activation 23 pour détecter le moment où a été effectuée l'administration de produit.

En variante, le premier capteur 21 d'orientation comprend également un capteur de force apte, lorsque le premier support 20 est fixé au dispositif de distribution 30, à détecter l'actionnement de moyens d'activation 23 du dispositif de distribution 30 et/ou la prise en main du dispositif de distribution 30. Avantageusement le premier support 20 comprend également un organe de contrôle apte à déclencher l'activation de l'ensemble de contrôle après que le premier capteur 21 d'orientation a détecté la prise en main du dispositif de distribution 30 par un utilisateur.

L'ensemble 1 comprend un deuxième support 10, 10' dont un premier mode de réalisation est illustré à la figure 2 ainsi qu'aux figures 6 et 7. Le deuxième support 10 comprend, un dispositif de fixation 12, 13 sur la tête de l'utilisateur, un deuxième capteur 11 d'orientation et une embase 17 de réception du deuxième capteur 11.

Dans le mode de réalisation tel qu'illustré par les figures 2, 6 et 7, le dispositif de fixation 12, 13 comprend des branches 13 configurées pour prendre appui sur les oreilles de l'utilisateur de manière à fixer le support de référence 10 sur la tête de l'utilisateur. Le support de référence 10 comprend en outre des patins 12 destinés à prendre appui de part et d'autre du nez de l'utilisateur, c'est-à-dire de part et d'autre de l'os formant le nez, et formant ainsi des moyens de centrage 12.

Le deuxième capteur 11 d'orientation est configuré pour mesurer une donnée relative à l'orientation du deuxième support 10 et notamment de l'embase 17 dans l'espace suivant trois axes de référence.

Le deuxième capteur 11 d'orientation est configuré pour communiquer avec le système de traitement afin de fournir, de préférence en temps réel, les données relatives à l'orientation du deuxième support 10 dans l'espace et donc à l'orientation de la tête de l'utilisateur dans l'espace lorsque le deuxième support 10 et notamment le dispositif de fixation 12, 13 est fixé sur la tête et/ou sur le visage de l'utilisateur. Les premier et deuxième capteurs 11, 21 d'orientation utilisés dans la présente invention sont des centrales inertielles, aussi appelées IMU pour Inertial Measurement Unit en anglais. Chaque centrale inertielle est composée d'au moins un accéléromètre et d'un gyroscope. L'accéléromètre permet de mesurer l'accélération linéaire d'un objet selon trois axes de mesure également appelés axes de référence du capteur et le gyroscope permet de mesurer la vitesse angulaire selon trois axes d'inclinaison. En ce qui concerne l'accéléromètre, les trois axes forment un repère orthonormé ainsi que trois plans orthogonaux. De manière générale, les axes d'inclinaison du gyroscope sont confondus avec les axes de mesure de l'accéléromètre dans une centrale inertielle.

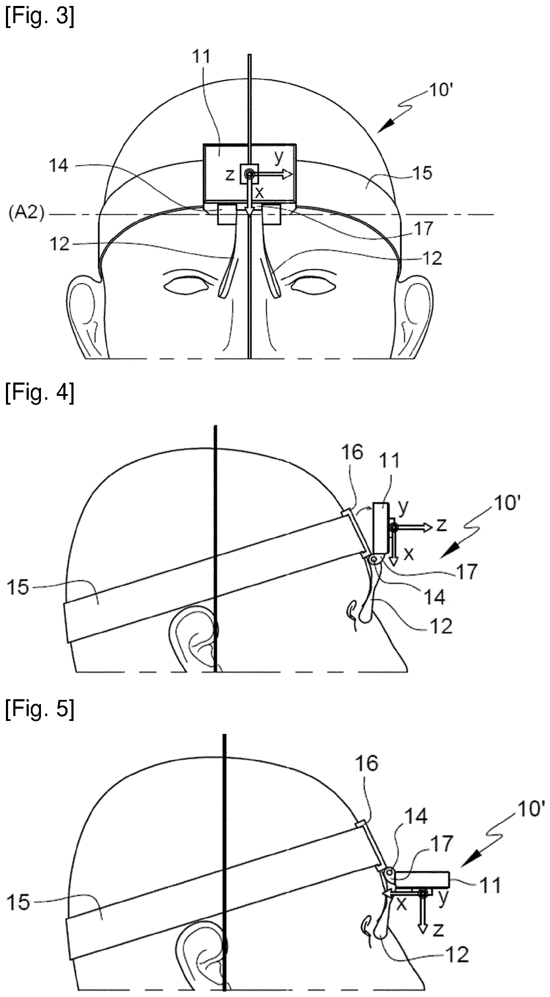

Dans la présente invention, pour que les capteurs puissent fournir des informations précises, il convient de régler les axes ou les plans orthogonaux par rapport à un repère. A cet effet, des éléments de réglage 14 de la position de l'embase 17 et donc du deuxième capteur 11 d'orientation par rapport au dispositif de fixation 12, 13 sont prévus sur le deuxième support 10. Les éléments de réglage 14 comprennent des éléments de pivotement du deuxième capteur 11 d'orientation selon un axe de pivotement (A2) perpendiculaire au plan sagittal de l'utilisateur, préférentiellement au plan sagittal de la tête de l'utilisateur, tel que visible sur la figure 3. Les éléments de réglage 14 peuvent être associés à des éléments de verrouillage de la position de l'embase 17 de sorte que l'embase 17 et le deuxième capteur 11 d'orientation puissent être immobilisés dans une position prédéterminée. Le réglage de l'embase 17sera décrit plus loin.

Les figures 3 à 5 illustrent un deuxième support 10' selon un deuxième mode de réalisation, dans lequel le dispositif de fixation 12, 15 comprend un bandeau élastique 15 formant un élément de plaquage 15 permettant d'être plaqué autour de la tête de l'utilisateur. Dans ce mode de réalisation, le bandeau élastique 15 comprend en outre des patins 12 similaires à ceux du premier mode de réalisation et une plaque 16 configurée pour prendre appui sur le front de l'utilisateur. Comme dans le premier mode de réalisation, le deuxième support 10' comprend également des éléments de réglage 14 et de verrouillage permettant de régler la position angulaire du deuxième capteur 11 d'orientation autour de l'axe de pivotement (A2).

Selon un mode de réalisation, le dispositif de fixation 12, 13, 15 comprend au moins trois zones distinctes d'appui sur le visage de l'utilisateur de façon à positionner le deuxième capteur 11 d'orientation et notamment ses axes de référence dans une position prédéterminée par rapport aux plans sagittal et/ou frontal de l'utilisateur, de préférence par rapport aux plans sagittal et/ou frontal de la tête de l'utilisateur.

Tel que visible aux figures 3 et 4, lorsque que le deuxième support 10' est fixé sur la tête de l'utilisateur, le deuxième capteur 11 d'orientation se trouve dans une orientation prédéterminée par rapport au plan sagittal et au plan frontal de l'utilisateur, préférentiellement par rapport au plan sagittal et au plan frontal de la tête de l'utilisateur : un premier plan formé par deux des axes de référence du deuxième capteur 11 d'orientation se trouve, lorsque le deuxième support 10' est fixé sur la tête de l'utilisateur, dans une orientation prédéterminée par rapport au plan sagittal de l'utilisateur, préférentiellement par rapport au plan sagittal de la tête de l'utilisateur, et un deuxième plan formé par deux des axes de référence du deuxième capteur 11 d'orientation et différent du premier plan, se trouve, lorsque le deuxième support 10' est fixé sur la tête de l'utilisateur, dans une orientation prédéterminée par rapport au plan frontal de l'utilisateur, préférentiellement par rapport au plan frontal de la tête de l'utilisateur.

Avantageusement, le premier plan s'étend parallèlement au plan sagittal de l'utilisateur, préférentiellement au plan sagittal de la tête de l'utilisateur. Plus précisément, dans l'exemple illustré, les trois axes de l'accéléromètre du deuxième capteur 11 d'orientation sont respectivement confondus avec les trois axes du gyroscope du même capteur. Les trois axes sont appelés ici axes x, y et z mais l'orientation de ces axes est arbitraire et peut être modifiée. Le plaquage du bandeau 15 sur la tête ou les branches 13 en appui sur les oreilles de l'utilisateur, ainsi que le positionnement des moyens de centrage 12 sur le nez assurent que le plan (x, z) formé par les axes x et z est sensiblement parallèle ou confondu avec le plan sagittal de l'utilisateur, préférentiellement avec le plan sagittal de la tête de l'utilisateur, lorsque le deuxième support 10, 10' est fixé sur la tête de l'utilisateur. Le plan formé par les axes x et z est ainsi sensiblement dans l'alignement de l'axe du nez. Après la mise en place du deuxième support 10, 10' sur la tête de l'utilisateur et avant la première utilisation du système de distribution de produit 100, il convient de régler le positionnement angulaire du deuxième capteur 11 d'orientation par rapport au plan frontal de l'utilisateur, préférentiellement par rapport au plan frontal de la tête de l'utilisateur. Pour ce faire et tel qu'illustré à la figure 4, l'utilisateur porte son regard loin devant lui, vers l'horizon, dans un plan sensiblement horizontal. Par la suite, au moyen des éléments de réglage 14, on fait pivoter le deuxième capteur 11 d'orientation jusqu'à ce que l'axe z soit sensiblement perpendiculaire au plan frontal de l'utilisateur, préférentiellement au plan frontal de la tête de l'utilisateur. Visuellement, il est possible d'apprécier le parallélisme entre le plan frontal de l'utilisateur, de préférence le plan frontal de la tête de l'utilisateur, et un plan formé par les deux autres axes x et y, ou encore la verticalité d'une des faces du deuxième capteur 11 d'orientation lorsque celui-ci a une forme de parallélépipède rectangulaire. Cette position du deuxième capteur 11 d'orientation correspond alors à une position de référence, encore appelée prédéterminée, où l'axe x du deuxième capteur 11 d'orientation est utilisé comme axe de référence pour le calcul par le système de traitement d'un angle sagittal (a) entre l'axe de distribution (A1) et le plan frontal de l'utilisateur, préférentiellement le frontal de la tête de l'utilisateur, et d'un angle frontal (b) entre l'axe de distribution (A1) et le plan sagittal de l'utilisateur, préférentiellement le plan sagittal de la tête de l'utilisateur. Le deuxième capteur 11 d'orientation est maintenu dans cette position par les éléments de réglage 14 et les éléments de verrouillage pour conserver le réglage durant toutes les utilisations ultérieures du système pour la distribution de produit. Dans cette position le deuxième plan (formé par les axes x et y) est dans une orientation prédéterminée par rapport au plan frontal de l'utilisateur, préférentiellement par rapport au plan frontal de la tête de l'utilisateur, et s'étend de préférence parallèlement au plan frontal de l'utilisateur, préférentiellement au plan frontal de la tête de l'utilisateur.

L'appellation des trois axes x, y et z ne constitue pas une limitation d'orientation des capteurs et est arbitraire. Selon une variante du réglage telle que visible à la figure 5, le deuxième capteur 11 d'orientation peut être positionné dans une autre position de référence ou prédéterminée dans laquelle le deuxième capteur 11 d'orientation a pivoté de 90° autour de l'axe de pivotement (A2) par rapport à la position illustrée dans la figure 4. Dans cette position, l'axe x est perpendiculaire au plan frontal de l'utilisateur, préférentiellement au plan frontal de la tête de l'utilisateur, et l'axe z est utilisé pour le calcul de l'angle sagittal (a) et de l'angle frontal (b).

Les figures 6 et 7 illustrent l'ensemble 1 de contrôle de la distribution de produit comprenant le premier support 20 et le deuxième support 10 selon le premier mode de réalisation illustré à la figure 2, le premier support 20 étant positionné selon une orientation prédéterminée par rapport au deuxième support 10. Tel que visible aux figures 6 et 7, l'orientation prédéterminée correspond à une orientation du premier support 20 où l'axe du dispositif de distribution (A1) forme avec le plan frontal de l'utilisateur un premier angle (a) qui est d'environ 30°, préférentiellement avec le plan frontal de la tête de l'utilisateur un premier angle (α) qui est d'environ 30°. Il s'agit donc d'un angle sagittal prédéterminé. Avec cette même orientation, l'axe du dispositif de distribution (A1) forme avec le plan sagittal de l'utilisateur un second angle (b) qui est d'environ 10°, préférentiellement avec le plan sagittal de la tête de l'utilisateur un second angle (β) qui est d'environ 10°. Il s'agit d'un angle frontal prédéterminé.

Le premier angle (a) est appelé angle sagittal car il est mesuré dans le plan sagittal de l'utilisateur, tandis que le deuxième angle (b) est appelé angle frontal car il est mesuré dans le plan frontal.

Le premier angle (α) est appelé angle sagittal car il est mesuré dans le plan sagittal de la tête de l'utilisateur, tandis que le deuxième angle (β) est appelé angle frontal car il est mesuré dans le plan frontal de la tête de l'utilisateur.

Selon une variante de l'invention, l'orientation prédéterminée souhaitée pour le premier support 20 par rapport au deuxième support 10, 10' et notamment à l'embase 17 est telle que l'angle sagittal est compris entre 25° et 35°, de préférence égal à 30° ; et que l'angle frontal est compris entre 5° et 15°, de préférence égal à 10°.

Les premier et deuxième capteurs 11, 21 d'orientation communiquent avec le système de traitement qui peut comprendre un module électronique tel qu'un circuit imprimé, ou PCB en anglais. Le module électronique comprend par exemple un microprocesseur contenant un logiciel de traitement des données fournies par le premier capteur 21 d'orientation et le deuxième capteur 11 d'orientation pour déterminer une information sur l'orientation du premier capteur 21 d'orientation, et donc du premier support 20 par rapport au deuxième capteur 11 d'orientation et donc au deuxième support 10, 10' et notamment à l'embase 17. Chaque premier et deuxième capteur 21, 11 d'orientation est apte à fournir des données relatives à son orientation par rapport au repère terrestre et le système de traitement est apte à calculer, en fonction desdites données, l'orientation du premier capteur 21 d'orientation dans le repère du deuxième capteur 11 d'orientation, c'est-à-dire par rapport au deuxième capteur 11 d'orientation. Par comparaison des angles relatifs entre le premier capteur 21 d'orientation et le deuxième capteur 11 d'orientation, le système de traitement calcule des angles entre le premier capteur 21 d'orientation et le deuxième capteur 11 d'orientation et notamment l'angle sagittal (a) et l'angle frontal (b) entre l'axe de distribution (A1) du produit et un axe de référence du deuxième capteur 11 d'orientation dont la position est définie par rapport aux plans sagittal et frontal de l'utilisateur, préférentiellement, par rapport aux plans sagittal et frontal de la tête de l'utilisateur, comme illustré en figures 6 et 7. En effet, l'orientation du premier support 20 et donc du premier capteur 21 d'orientation par rapport au dispositif de distribution 30 et notamment à l'axe de distribution (A1) étant connue, de même que l'orientation du deuxième support 10, 10' et donc du deuxième capteur 11 d'orientation par rapport aux plans sagittal et frontal de l'utilisateur, préférentiellement par rapport aux plan sagittal et frontal de la tête de l'utilisateur, il est possible de déterminer les angles sagittal et frontal. Grace au deuxième support 10, 10', l'orientation de l'axe de référence du deuxième capteur 11 d'orientation par rapport au nez de l'utilisateur est répétable, quelle que soit la position de l'utilisateur.

Avantageusement le système de traitement est embarqué sur le deuxième support 10, 10' ou sur le premier support 20. Selon une variante de l'invention, le système de traitement est séparé du premier support 20 et du deuxième support 10, 10'. Il peut, par exemple, être intégré dans un téléphone mobile multifonction, également appelé smartphone ou tout autre appareil électronique convenable.

Avantageusement, le système de traitement est apte à verrouiller les moyens d'activation 23 selon l'information fournie sur l'orientation du premier support 20 par rapport au deuxième support 10, 10'. Cette configuration permet de verrouiller les moyens d'activation 23 lorsque le premier support 20 n'est pas positionné selon l'orientation prédéterminée.

Dans les modes de réalisation décrits, lorsque le deuxième support 10, 10' est fixé sur la tête de l'utilisateur, le deuxième capteur 11 d'orientation se trouve dans une orientation prédéterminée par rapport au plan sagittal et au plan frontal de l'utilisateur, préférentiellement par rapport au plan sagittal et au plan frontal de la tête de l'utilisateur. Toutefois, dans d'autres modes de réalisation de l'invention, le deuxième support est configuré pour être fixé sur le front ou le nez de l'utilisateur, de sorte que le deuxième capteur 11 d'orientation se trouve dans une orientation prédéterminée par rapport au moins à l'un des plans sagittal et frontal de l'utilisateur, préférentiellement par rapport au moins l'un des plans sagittal et frontal de la tête de l'utilisateur. Ces modes de réalisations permettent de déterminer au moins un des angles sagittal et frontal et donc de disposer d'un ensemble de contrôle fiable et efficace pour détecter l'orientation du dispositif de distribution par rapport à une narine de l'utilisateur.

Un procédé de contrôle de la distribution de produit dans une cavité nasale d'un utilisateur à l'aide de l'ensemble 1 de contrôle de la distribution de produit tel que décrit précédemment et comprenant, par exemple, un deuxième support 10 selon le premier mode de réalisation de l'invention sera décrit ci-dessous. Le procédé comprend les étapes suivantes :
étape 1 : le premier support 20 est fixé à un dispositif de distribution 30 de produit par voie nasale.
étape 2 : le deuxième support 10, 10' est fixé sur le nez ou le front d'un utilisateur de sorte qu'un premier plan formé par deux des axes de référence du deuxième capteur 11 d'orientation se trouve dans une orientation prédéterminée par rapport à un plan sagittal de l'utilisateur, préférentiellement par rapport au plan sagittal de la tête de l'utilisateur, ou par rapport à un plan frontal de l'utilisateur, préférentiellement par rapport au plan frontal de la tête de l'utilisateur. Avantageusement au cours de l'étape 2 le deuxième support 10, 10' est fixé sur la tête de l'utilisateur de sorte que le premier plan est parallèle au plan sagittal de l'utilisateur, préférentiellement au plan sagittal de la tête de l'utilisateur. Par exemple, les branches 13 du dispositif de fixation 12, 13 sont mises en appui sur les oreilles de l'utilisateur et les patins 12 sont mis en appui de part et d'autre du nez de l'utilisateur. Avantageusement, au cours de l'étape 2, la position de l'embase 17 est réglée à l'aide des éléments de réglage 14 pour faire pivoter l'embase 17 et donc le deuxième capteur 11 selon l'axe de pivotement (A2) de sorte que le deuxième plan est parallèle au plan frontal de l'utilisateur, préférentiellement au plan frontal de la tête de l'utilisateur. A cet effet l'utilisateur porte son regard vers l'horizon dans un plan de vision sensiblement horizontal et l'utilisateur ou un praticien fait pivoter l'embase 17 de façon à positionner l'embase 17 verticalement par rapport au sol, puis verrouille la position de l'embase 17 à l'aide des éléments de verrouillage.
étape 3 : le premier capteur 21 d'orientation et le deuxième capteur 11 d'orientation mesurent des données. Avantageusement, la mesure est déclenchée seulement lorsque la prise en main du dispositif de distribution 30 par un utilisateur est détectée.
étape 4 : le système de traitement des données détermine une information sur l'orientation du premier support 20 par rapport au deuxième support 10, 10' en fonction des données mesurées par le premier capteur 21 d'orientation et le deuxième capteur 11 d'orientation.

Lorsqu'il est par exemple mis en œuvre pour chaque dispositif de distribution d'un ensemble de dispositifs de distribution à tester lors « d'études utilisateur », un tel procédé aide à identifier le dispositif de distribution qui permet d'obtenir le positionnement requis pour une distribution de produit efficace dans la zone cible.

Avantageusement, préalablement à l'étape 1, le procédé comprend une étape d'initialisation des premier et deuxième capteurs 21, 11 d'orientation au cours de laquelle les premier et deuxième capteurs 21, 11 d'orientation et notamment les premier et deuxième supports 20, 10, 10' sont positionnés parallèlement l'un à l'autre de sorte que les axes de références respectifs des capteurs sont parallèles pour définir une référence commune pour la mesure de l'orientation des capteurs par la suite. Ensuite le fonctionnement des premier et deuxième capteurs 21, 11 d'orientation est activé ou les capteurs sont réinitialisés pour mémoriser cette référence commune.

L'invention n'est pas limitée aux modes de réalisation présentés et d'autres modes de réalisation apparaîtront clairement à l'homme du métier. Il est notamment possible d'utiliser un premier moyen de signalisation pour émettre un premier signal lorsque le premier support 20 est positionné selon l'orientation prédéterminée souhaitée par rapport au deuxième support 10, 10'. Il est également possible d'utiliser un second moyen de signalisation pour émettre un second signal, continu ou intermittent, aussi longtemps que le premier support 20 n'est pas positionné selon l'orientation prédéterminée. Ainsi, après l'étape 4, le système de traitement est par exemple apte à transmettre en fonction de l'orientation du premier support 20 par rapport au deuxième support 10, 10', des informations pour assister l'utilisateur dans le positionnement souhaité du dispositif de distribution 30, par exemple au cours d'une utilisation pendant une phase clinique, ou lors d'une utilisation à domicile.

### Liste de références

1 : ensemble
10, 10' : deuxième support
11 : deuxième capteur d'orientation
12 : moyens de centrage, patins
13 : branches
14 : éléments de réglage
15 : élément de plaquage, bandeau
16 : plaque
17 : embase
20 : premier support
21 : premier capteur d'orientation
22 : moyens de fixation
23 : moyens d'activation
30 : dispositif de distribution
31 : réservoir de produit
32 : tête de distribution
100 : système de distribution de produit
A1 : axe du dispositif, axe de distribution
A2 : axe de pivotement
a : premier angle, angle sagittal
b : second angle, angle frontal

## Revendications

1. Ensemble (1) de contrôle de la distribution de produit dans une cavité nasale d'un utilisateur comprenant :
- un premier support (20) configuré pour être fixé à un dispositif de distribution (30) de produit par voie nasale, et comprenant un premier capteur (21) d'orientation configuré pour mesurer une donnée relative à l'orientation du premier support dans l'espace,
- un deuxième support (10, 10') configuré pour être fixé sur la tête d'un utilisateur, et comprenant un deuxième capteur (11) d'orientation configuré pour mesurer une donnée relative à l'orientation du deuxième support (10, 10') dans l'espace suivant trois axes de référence, et
- un système de traitement d'information configuré pour recevoir les données mesurées par le premier capteur (21) d'orientation et le deuxième capteur (11) d'orientation et pour déterminer une information sur l'orientation du premier support (21) par rapport au deuxième support (10,10') en fonction desdites données mesurées, et **caractérisé en ce que** le deuxième support (10, 10') est configuré pour être fixé sur le front ou le nez de l'utilisateur, de façon qu'un premier plan formé par deux des axes de référence du deuxième capteur (11) d'orientation se trouve, lorsque le deuxième support (10, 10') est fixé sur la tête de l'utilisateur, dans une orientation prédéterminée par rapport à un plan sagittal de l'utilisateur ou par rapport à un plan frontal de l'utilisateur.

2. Ensemble (1) selon la revendication précédente, dans lequel l'orientation prédéterminée du premier plan, correspond à une orientation parallèle au plan sagittal de l'utilisateur ou au plan frontal de l'utilisateur.

3. Ensemble (1) selon la revendication 1 ou 2, dans lequel le deuxième support (10, 10') est configuré pour être fixé sur le front ou le nez de l'utilisateur, de façon :
- que le premier plan se trouve dans une orientation prédéterminée par rapport au plan sagittal de l'utilisateur, et
- qu'un deuxième plan formé par deux des axes de référence du deuxième capteur (11) d'orientation et différent du premier plan, se trouve dans une orientation prédéterminée par rapport à un plan frontal de l'utilisateur.

4. Ensemble (1) selon l'une quelconque des revendications précédentes, dans lequel le deuxième capteur (11) d'orientation comprend un accéléromètre configuré pour mesurer l'accélération linéaire suivant trois axes de mesure, les axes de mesure correspondant aux axes de référence du deuxième capteur (11) d'orientation.

5. Ensemble (1) selon l'une quelconque des revendications précédentes, dans lequel le deuxième support (10, 10') comprend :
- un dispositif de fixation (12, 13, 15) sur la tête de l'utilisateur,
- une embase (17) de réception du deuxième capteur (11) d'orientation,
- des éléments de réglage (14) de la position de l'embase (17), par rapport au dispositif de fixation (12, 13, 15), et des éléments de verrouillage de la position de l'embase (17) de sorte que la position du deuxième capteur (11) d'orientation par rapport au plan sagittal puisse être réglée et verrouillée une fois que le dispositif de fixation (12, 13, 15) est fixé sur la tête d'un utilisateur,
les éléments de réglage (14) comprenant de préférence des éléments de pivotement de l'embase (17) selon un axe de pivotement (A2) perpendiculaire au plan sagittal de l'utilisateur.

6. Ensemble (1) selon l'une quelconque des revendications 1 à 4,
dans lequel le deuxième support (10, 10') comprend un dispositif de fixation (12, 13, 15) sur la tête de l'utilisateur et une embase (17) de réception du deuxième capteur (11) d'orientation, le dispositif de fixation (12, 13, 15) comprenant au moins trois zones d'appui sur le visage de l'utilisateur.

7. Ensemble (1) selon la revendication précédente, dans lequel le dispositif de fixation (12, 13, 15) comprend :
- des moyens de centrage (12) sur le nez de l'utilisateur, par exemple des patins (12) prenant appui de part et d'autre du nez, et
- des branches (13) configurées pour prendre appui sur les oreilles de l'utilisateur ou un élément de plaquage (15) contre le front, par exemple un bandeau élastique (15) configuré pour être plaqué autour de la tête de l'utilisateur.

8. Ensemble (1) selon l'une quelconque des revendications précédentes, dans lequel le premier capteur (21) d'orientation est configuré pour mesurer une donnée relative à l'orientation du premier support dans l'espace suivant trois axes de référence, le premier support (20) étant configuré pour que, lorsqu'il est fixé au dispositif de distribution (30), un des axes de référence du premier capteur (21) d'orientation soit dans une orientation prédéterminée par rapport à un axe de distribution (A1) du dispositif de distribution (30), de préférence pour qu'un des axes de référence du premier capteur (21) d'orientation soit parallèle, de préférence encore confondu avec l'axe de distribution (A1).

9. Ensemble (1) selon l'une quelconque des revendications précédentes, dans lequel le premier support (20) comprend des moyens d'activation (23) configurés pour être actionnés par les doigts d'une personne et pour coopérer avec le dispositif de distribution (30) afin de distribuer une dose de produit et dans lequel le premier capteur (21) d'orientation est configuré pour détecter l'actionnement des moyens d'activation (23) en plus de l'orientation du premier support.

10. Ensemble (1) selon l'une quelconque des revendications précédentes comprenant un moyen de signalisation, dit premier moyen de signalisation, configuré pour émettre un premier signal lorsque le système de traitement détermine que le premier support (20) est positionné selon une orientation prédéterminée par rapport au deuxième support (10, 10').

11. Ensemble (1) selon l'une quelconque des revendications précédentes comprenant un moyen de signalisation, dit second moyen de signalisation, configuré pour émettre un second signal, continu ou intermittent, aussi longtemps que le système de traitement détermine que le premier support (20) n'est pas positionné selon une orientation prédéterminée par rapport au deuxième support (10, 10').

12. Ensemble (1) selon l'une quelconque des revendications précédentes dans lequel le système de traitement est configuré pour déterminer, lorsque le deuxième support (10, 10') est fixé sur la tête de l'utilisateur, en fonction des données mesurées par le premier capteur (21) d'orientation et le deuxième capteur (11) d'orientation :
- un premier angle (a) formé entre un axe de distribution (A1) du dispositif de distribution (30) et le plan frontal, et/ou
- un deuxième angle (b) formé entre un axe de distribution (A1) du dispositif de distribution (30) et le plan sagittal.

13. Système de distribution de produit (100) dans une cavité nasale d'un utilisateur comprenant un dispositif de distribution (30) de produit par voie nasale et un ensemble (1) selon l'une quelconque des revendications précédentes.

14. Procédé de contrôle de la distribution de produit dans une cavité nasale d'un utilisateur à l'aide d'un ensemble (1) selon l'une quelconque des revendications 1 à 12 comprenant les étapes suivantes :
- étape 1 : le premier support (20) est fixé à un dispositif de distribution (30) de produit par voie nasale,
- étape 2 : le deuxième support (10, 10') est fixé sur le front ou le nez d'un utilisateur de sorte qu'un premier plan formé par deux des axes de référence du deuxième capteur (11) d'orientation se trouve dans une orientation prédéterminée par rapport à un plan sagittal de l'utilisateur ou par rapport à un plan frontal de l'utilisateur,
- étape 3 : le premier capteur (21) d'orientation et le deuxième capteur (11) d'orientation mesurent des données, et
- étape 4 : le système de traitement d'information détermine une information sur l'orientation du premier support (20) par rapport au deuxième support (10, 10') en fonction des données mesurées par le premier capteur (21) d'orientation et le deuxième capteur (11) d'orientation.

15. Procédé selon la revendication précédente, dans lequel lors de l'étape 2 de fixation du deuxième support (10, 10') sur la tête de l'utilisateur, le premier plan est dans une orientation prédéterminée par rapport au plan sagittal de l'utilisateur, et un réglage du positionnement angulaire du deuxième capteur (11) d'orientation est effectué de sorte qu'un deuxième plan formé par deux des axes de référence du deuxième capteur (11) d'orientation et différent du premier plan, se trouve dans une orientation prédéterminée par rapport à un plan frontal de l'utilisateur.

## Patentansprüche

1. Vorrichtung (1) zur Steuerung der Abgabe eines Produkts in eine Nasenhöhle eines Benutzers, aufweisend:
- eine erste Halterung (20), die konfiguriert ist, um an einer Vorrichtung (30) zur nasalen Abgabe eines Produkts befestigt zu werden, und einen ersten Orientierungssensor (21) aufweist, der konfiguriert ist, Daten bezüglich der Orientierung der ersten Halterung im Raum zu messen,
- eine zweite Halterung (10, 10'), die konfiguriert ist, am Kopf eines Benutzers befestigt zu werden, und einen zweiten Orientierungssensor (11) aufweist, der konfiguriert ist, Daten bezüglich der Orientierung der zweiten Halterung (10, 10') im Raum entlang drei Referenzachsen zu messen, und
- ein Informationsverarbeitungssystem, das konfiguriert ist, die vom ersten Orientierungssensor (21) und vom zweiten Orientierungssensor (11) gemessenen Daten zu empfangen und anhand dieser gemessenen Daten Informationen über die Orientierung der ersten Halterung (20) relativ zur zweiten Halterung (10, 10') zu ermitteln, und **dadurch gekennzeichnet, dass** die zweite Halterung (10, 10') konfiguriert ist, an der Stirn oder Nase des Benutzers befestigt zu werden, sodass eine erste Ebene, die durch zwei der Referenzachsen des zweiten Orientierungssensors (11) gebildet wird, sich, wenn die zweite Halterung (10, 10°) am Kopf des Benutzers befestigt ist, in einer vorbestimmten Ausrichtung in Bezug auf eine Sagittalebene des Benutzers oder in Bezug auf eine Frontalebene des Benutzers befindet.

2. Vorrichtung (1) nach dem vorhergehenden Anspruch, wobei die vorbestimmte Ausrichtung der ersten Ebene einer Ausrichtung parallel zur Sagittalebene des Benutzers oder zur Frontalebene des Benutzers entspricht.

3. Vorrichtung (1) nach Anspruch 1 oder 2, wobei die zweite Halterung (10, 10°) konfiguriert ist, an der Stirn oder Nase des Benutzers befestigt zu werden, sodass:
- die erste Ebene in einer vorbestimmten Ausrichtung in Bezug auf die Sagittalebene des Benutzers liegt und
- eine zweite Ebene, die durch zwei der Referenzachsen des zweiten Orientierungssensors (11) gebildet wird und sich von der ersten Ebene unterscheidet, in einer vorbestimmten Ausrichtung in Bezug auf eine Frontalebene des Benutzers liegt.

4. Vorrichtung (1) nach einem der vorhergehenden Ansprüche, wobei der zweite Orientierungssensor (11) einen Beschleunigungsmesser aufweist, der konfiguriert ist, die lineare Beschleunigung entlang drei Messachsen zu messen, wobei die Messachsen den Referenzachsen des zweiten Orientierungssensors (11) entsprechen.

5. Vorrichtung (1) nach einem der vorhergehenden Ansprüche, wobei die zweite Halterung (10, 10') aufweist:
- eine Befestigungsvorrichtung (12, 13, 15) am Kopf des Benutzers,
- eine Basis (17) zur Aufnahme des zweiten Orientierungssensors (11),
- Elemente (14) zum Einstellen der Position der Basis (17) in Bezug auf die Befestigungsvorrichtung (12, 13, 15) und Elemente zum Verriegeln der Position der Basis (17), so dass die Position des zweiten Orientierungssensors (11) in Bezug auf die Sagittalebene eingestellt und verriegelt werden kann, sobald die Befestigungsvorrichtung (12, 13, 15) am Kopf eines Benutzers befestigt ist, wobei
die Einstellelemente (14) vorzugsweise Elemente zum Schwenken der Basis (17) um eine Schwenkachse (A2) senkrecht zur Sagittalebene des Benutzers aufweisen.

6. Vorrichtung (1) nach einem der Ansprüche 1 bis 4, wobei die zweite Halterung (10, 10°) eine Befestigungsvorrichtung (12, 13, 15) am Kopf des Benutzers und eine Basis (17) zur Aufnahme des zweiten Orientierungssensors (11) aufweist, wobei die Befestigungsvorrichtung (12, 13, 15) mindestens drei Auflagebereiche auf dem Gesicht des Benutzers aufweist.

7. Vorrichtung (1) nach dem vorhergehenden Anspruch, wobei die Befestigungsvorrichtung (12, 13, 15) aufweist:
- Mittel zum Zentrieren (12) auf der Nase des Benutzers, beispielsweise Polster (12), die auf beiden Seiten der Nase aufliegen, und
- Bügel (13), die konfiguriert sind, auf den Ohren des Benutzers aufzuliegen, oder ein Element (15) zum Andrücken an die Stirn, beispielsweise ein elastisches Band (15), das konfiguriert ist, um den Kopf des Benutzers herum angelegt zu werden.

8. Vorrichtung (1) nach einem der vorhergehenden Ansprüche, wobei der erste Orientierungssensor (21) konfiguriert ist, Daten bezüglich der Orientierung der ersten Halterung im Raum entlang drei Referenzachsen zu messen, wobei die erste Halterung (20) so konfiguriert ist, dass, wenn er an der Abgabevorrichtung (30) befestigt ist, eine der Referenzachsen des ersten Orientierungssensors (21) in einer vorbestimmten Ausrichtung in Bezug auf eine Abgabeachse (A1) der Abgabevorrichtung (30) ist, vorzugsweise so, dass eine der Referenzachsen des ersten Orientierungssensors (21) parallel zu der Abgabeachse (A1) ist, vorzugsweise sogar mit dieser zusammenfällt.

9. Vorrichtung (1) nach einem der vorhergehenden Ansprüche, wobei die erste Halterung (20) Betätigungsmittel (23) aufweist, die konfiguriert sind, durch die Finger einer Person betätigt zu werden und mit der Abgabevorrichtung (30) zusammenwirken, um eine Produktdosis abzugeben, und wobei der erste Orientierungssensor (21) konfiguriert ist, zusätzlich zur Orientierung der ersten Halterung die Betätigung der Betätigungsmittel (23) zu erfassen.

10. Vorrichtung (1) nach einem der vorhergehenden Ansprüche, die eine Signaleinrichtung, die sogenannte erste Signaleinrichtung, aufweist, die konfiguriert ist, ein erstes Signal auszugeben, wenn das Verarbeitungssystem feststellt, dass die erste Halterung (20) in einer vorbestimmten Ausrichtung in Bezug auf die zweite Halterung (10, 10") positioniert ist.

11. Vorrichtung (1) nach einem der vorhergehenden Ansprüche, aufweisend eine Signaleinrichtung, die als zweite Signaleinrichtung bezeichnet wird und konfiguriert ist, ein zweites Signal, entweder kontinuierlich oder intermittierend, auszusenden, solange das Verarbeitungssystem feststellt, dass die erste Halterung (20) nicht in einer vorbestimmten Ausrichtung in Bezug auf die zweite Halterung (10, 10") positioniert ist.

12. Vorrichtung (1) nach einem der vorhergehenden Ansprüche, wobei das Verarbeitungssystem so konfiguriert ist, dass es, wenn die zweite Halterung (10, 10") am Kopf des Benutzers befestigt ist, auf der Grundlage der vom ersten Orientierungssensor (21) und vom zweiten Orientierungssensor (11) gemessenen Daten Folgendes bestimmt:
- einen ersten Winkel (a), der zwischen einer Abgabeachse (A1) der Abgabevorrichtung (30) und der Frontalebene gebildet wird, und/oder
- einen zweiten Winkel (b), der zwischen einer Abgabeachse (A1) der Abgabevorrichtung (30) und der Sagittalebene gebildet wird.

13. System (100) zur Abgabe eines Produkts in eine Nasenhöhle eines Benutzers, aufweisend eine Vorrichtung (30) zur nasalen Abgabe eines Produkts und eine Vorrichtung (1) gemäß einem der vorstehenden Ansprüche.

14. Verfahren zur Steuerung der Abgabe eines Produkts in eine Nasenhöhle eines Benutzers unter Verwendung einer Vorrichtung (1) gemäß einem der Ansprüche 1 bis 12, aufweisend die folgenden Schritte:
- Schritt 1: Die erste Halterung (20) wird an einer Vorrichtung (30) zur nasalen Abgabe eines Produkts befestigt.
- Schritt 2: Die zweite Halterung (10, 10') wird an der Stirn oder Nase eines Benutzers befestigt, sodass eine erste Ebene, die durch zwei der Referenzachsen des zweiten Orientierungssensors (11) gebildet wird, in einer vorbestimmten Ausrichtung in Bezug auf eine Sagittalebene des Benutzers oder in Bezug auf eine Frontalebene des Benutzers liegt.
- Schritt 3: Der erste Orientierungssensor (21) und der zweite Orientierungssensor (11) messen Daten, und
- Schritt 4: Das Informationsverarbeitungssystem ermittelt Informationen über die Ausrichtung der ersten Halterung (20) in Bezug auf die zweite Halterung (10, 10') auf der Grundlage der vom ersten Orientierungssensor (21) und vom zweiten Orientierungssensor (11) gemessenen Daten.

15. Verfahren nach dem vorhergehenden Anspruch, wobei in Schritt 2 beim Befestigen der zweiten Halterung (10, 10°) am Kopf des Benutzers die erste Ebene in einer vorbestimmten Ausrichtung in Bezug auf die Sagittalebene des Benutzers ist und eine Einstellung der Winkelpositionierung des zweiten Orientierungssensors (11) so vorgenommen wird, dass eine zweite Ebene, die durch zwei der Referenzachsen des zweiten Orientierungssensors (11) gebildet wird und sich von der ersten Ebene unterscheidet, in einer vorbestimmten Ausrichtung in Bezug auf eine Frontalebene des Benutzers ist.

## Claims

1. Assembly (1) for controlling the dispensing of a product into a nasal cavity of a user, comprising:
- a first holder (20) configured to be fastened to a product dispensing device (30) for nasal pathway, and comprising a first orientation sensor (21) configured to measure a data relating to the orientation of the first holder in space,
- a second holder (10, 10') configured to be fastened to the head of a user, and comprising a second orientation sensor (11) configured to measure a data relating to the orientation of the second holder (10, 10') in space along three reference axes, and
- an information processing system configured to receive the data measured by the first orientation sensor (21) and the second orientation sensor (11) and to determine an information about the orientation of the first holder (21) with respect to the second holder (10, 10') according to said measured data,
**characterised in that** the second holder (10, 10') is configured to be fastened to the user's forehead or nose, so that a first plane formed by two of the reference axes of the second orientation sensor (11) is located, when the second holder (10, 10') is fastened to the user's head, in a predetermined orientation with respect to a sagittal plane of the user or with respect to a frontal plane of the user.

2. Assembly (1) according to the preceding claim, wherein the predetermined orientation of the first plane corresponds to an orientation parallel to the sagittal plane of the user or to the frontal plane of the user.

3. Assembly (1) according to claim 1 or 2, wherein the second holder (10, 10') is configured to be fastened to the user's forehead or nose, so that:
- the first plane is located in a predetermined orientation with respect to the sagittal plane of the user, and
- a second plane, formed by two of the reference axes of the second orientation sensor (11) and different from the first plane, is located in a predetermined orientation with respect to a frontal plane of the user.

4. Assembly (1) according to any one of the preceding claims, wherein the second orientation sensor (11) comprises an accelerometer configured to measure the linear acceleration along three measurement axes, the measurement axes corresponding to the reference axes of the second orientation sensor (11).

5. Assembly (1) according to any one of the preceding claims, wherein the second holder (10, 10') comprises:
- a fastening device (12, 13, 15) on the user's head,
- a base (17) for receiving the second orientation sensor (11),
- adjustment elements (14) for adjusting the position of the base (17), with respect to the fastening device (12, 13, 15), and locking elements for locking the position of the base (17) so that the position of the second orientation sensor (11) with respect to the sagittal plane can be adjusted and locked once the fastening device (12, 13, 15) is fastened to the head of a user,
the adjustment elements (14) preferably comprising pivoting elements for pivoting the base (17) about a pivoting axis (A2) perpendicular to the sagittal plane of the user.

6. Assembly (1) according to any of the claims 1 to 4, wherein the second holder (10, 10') comprises a fastening device (12, 13, 15) on the user's head and a base (17) for receiving the second orientation sensor (11), the fastening device (12, 13, 15) comprising at least three bearing zones on the user's face.

7. Assembly (1) according to the preceding claim, wherein the fastening device (12, 13, 15) comprises:
- centring means (12) for centring on the user's nose, for example pads (12) bearing on each side of the nose, and
- branches (13) configured to bear against the user's ears or an pressing element (15) for pressing against the forehead, for example an elastic headband (15) configured to be pressed around the user's head.

8. Assembly (1) according to any one of the preceding claims, wherein the first orientation sensor (21) is configured to measure a data relating to the orientation of the first holder in space along three reference axes, the first holder (20) being configured so that, when it is fastened to the dispensing device (30), one of the reference axes of the first orientation sensor (21) is in a predetermined orientation with respect to a dispensing axis (A1) of the dispensing device (30), preferably so that one of the reference axes of the first orientation sensor (21) is parallel to, more preferably coincides with, the dispensing axis (A1).

9. Assembly (1) according to any one of the preceding claims, wherein the first holder (20) comprises activation means (23) configured to be actuated by the fingers of a person and to cooperate with the dispensing device (30) in order to dispense a dose of product and wherein the first orientation sensor (21) is configured to detect the actuation of the activation means (23) in addition to the orientation of the first holder.

10. Assembly (1) according to any one of the preceding claims comprising signalling means, called first signalling means, configured to emit a first signal when the processing system determines that the first holder (20) is positioned in a predetermined orientation with respect to the second holder (10, 10').

11. Assembly (1) according to any one of the preceding claims comprising signalling means, called second signalling means, configured to emit a second continuous or intermittent signal, for as long as the processing system determines that the first holder (20) is not positioned in a predetermined orientation with respect to the second holder (10, 10').

12. Assembly (1) according to any one of the preceding claims, wherein the processing system is configured to determine, when the second holder (10, 10') is fastened to the user's head, according to the data measured by the first orientation sensor (21) and the second orientation sensor (11):
- a first angle (a) formed between a dispensing axis (A1) of the dispensing device (30) and the frontal plane, and/or
- a second angle (b) formed between a dispensing axis (A1) of the dispensing device (30) and the sagittal plane.

13. System (100) for the dispensing of a product into a nasal cavity of a user comprising a product dispensing device (30) for nasal pathway and an assembly (1) according to any one of the preceding claims.

14. Method for controlling the dispensing of a product into a nasal cavity of a user using an assembly (1) according to any one of claims 1 to 12 comprising the following steps:
- step 1: the first holder (20) is fastened to a product dispensing device (30) for nasal pathway,
- step 2: the second holder (10, 10') is fastened to the forehead or nose of a user, so that a first plane formed by two of the reference axes of the second orientation sensor (11) is located in a predetermined orientation with respect to a sagittal plane of the user, or with respect to a frontal plane of the user,
- step 3: the first orientation sensor (21) and the second orientation sensor (11) measure data, and
- step 4: the information processing system determines an information about the orientation of the first holder (20) with respect to the second holder (10, 10') according to the data measured by the first orientation sensor (21) and the second orientation sensor (11).

15. Method according to the preceding claim, wherein during step 2 of fastening the second holder (10, 10') to the user's head, the first plane is in a predetermined orientation with respect to the sagittal plane of the user, and the angular positioning of the second orientation sensor (11) is adjusted so that a second plane, formed by two of the reference axes of the second orientation sensor (11) and different from the first plane, is located in a predetermined orientation with respect to a frontal plane of the user
